# EUROPEAN PATENT SPECIFICATION

(11) **EP 3 506 905 B1**
(45) Date of publication and mention of the grant of the patent: **20.03.2024**
(21) Application number: 17761723.0
(22) Date of filing: 24.08.2017
(51) Int. Cl.: A61K 31/55, A61P 35/00, A61K 45/06, A61K 31/573

(54) **DOSAGE REGIMEN FOR THE TREATMENT OF SOLID TUMORS**
DOSIERSCHEMA ZUR BEHANDLUNG VON SOLIDEN TUMOREN
SCHÉMA POSOLOGIQUE POUR LE TRAITEMENT DE TUMEURS SOLIDES

(30) Priority: 31.08.2016 US 201662381911 P
(43) Date of publication of application: 10.07.2019
(73) Proprietor: Eli Lilly and Company, Indianapolis, IN 46285 (US)
(72) Inventor: BENHADJI, Karim Adnane, Indianapolis IN 46206-6288 (US); YUEN, Eunice Soek Mun, Indianapolis IN 46206-6288 (US)
(74) Representative: Carpmaels & Ransford LLP
(86) International application number: PCT/US2017/048308
(87) International publication number: WO 2018/044662

(56) References cited:
- WO-A1-2013/016081
- WO-A1-2016/168014
- Christophe Massard: "First-in-human study of LY3039478, a Notch signaling inhibitor in advanced or metastatic cancer", Journal of Clinical Oncology: Vol 33, No 15_suppl, 1 May 2015 (2015-05-01), XP055417831, Retrieved from the Internet: URL:http://ascopubs.org/doi/abs/10.1200/jc o.2015.33.15_suppl.2533 [retrieved on 2017-10-20]
- Eunice Yuen: "Population pharmacokinetics and pharmacodynamics for an oral Notch inhibitor, LY3039478, in the first-in-man study", Cancer Research, 1 July 2016 (2016-07-01), XP055417836, Retrieved from the Internet: URL:http://cancerres.aacrjournals.org/cont ent/76/14_Supplement/CT048 [retrieved on 2017-10-20]
- AZARO ANALÍA ET AL: "Phase 1 study of 2 high dose intensity schedules of the pan-Notch inhibitor crenigacestat (LY3039478) in combination with prednisone in patients with advanced or metastatic cancer", INVESTIGATIONAL NEW DRUGS, vol. 39, no. 1, 11 September 2020 (2020-09-11), pages 193-201, XP037353940, ISSN: 0167-6997, DOI: 10.1007/S10637-020-00944-Z
- Bender Mark H: "Novel inhibitor of Notch signaling for the treatment of cancer. | Cancer Research | American Association for Cancer Research", Cancer Res 73 (8), 1 April 2013 (2013-04-01), page Abstract 1131, XP093021940, Retrieved from the Internet: URL:https://aacrjournals.org/cancerres/art icle/73/8_Supplement/1131/586729/Abstract- 1131-Novel-inhibitor-of-Notch-signaling [retrieved on 2023-02-08]

## Description

Notch signaling plays an important role during development and tissue homeostasis. Dysregulation of Notch signaling due to mutation, amplification, or overexpression of ligands and/or receptors, is implicated in a number of malignancies. Inhibition of Notch signaling through inhibition of gamma secretase cleavage of the Notch signaling pathway is a potential target for the development of cancer therapeutics. 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide, or a pharmaceutically acceptable salt or hydrate thereof, and methods of making and using this compound, including for the treatment of T-cell acute lymphoblastic leukemia, acute lymphoblastic leukemia, acute myelogenous leukemia, chronic myelogenous leukemia, erythroleukemia, breast cancer, ovarian cancer, melanoma, lung cancer, pancreatic cancer, glioblastoma, colorectal cancer, head and neck cancer, cervical cancer, prostate cancer, liver cancer, squamous cell carcinoma (oral), skin cancer and medulloblastoma are disclosed in WO 2013/016081. 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide, or a pharmaceutically acceptable salt or hydrate thereof, is being investigated in a phase 1 clinical trial and expansion cohorts having a defined molecular pathway alteration, or a tissue based malignant tumor, and in combination with other specifically identified anticancer agents against specified tumor types and in a clinical trial in patients with T-cell acute lymphoblastic leukemia or T-cell lymphoblastic lymphoma (T-ALL/T-LBL).

The most serious toxicities associated with gamma secretase inhibitors targeting the Notch pathway for treating cancer are gastrointestinal toxicities such as diarrhea including mucoid enteropathy or mucoid gastroenteropathy. Rapid differentiation of progenitor cells into secretory goblet cells in the intestinal crypts occurs after administration of gamma secretase inhibitors. Notch signaling is required to maintain the normal architecture of the intestinal epithelium. *In vivo* models have been used to evaluate methods of ameliorating the gastrointestinal toxicity by administration of gamma secretase inhibitors with intermittent dosing and co-administration of corticosteroids Bender et al., Cancer Res., 2013, 73(8) Supplement, Abstract 1131. While meeting with some success, suitable efficacy against solid tumor cancers with acceptable gastrointestinal toxicities remains elusive. (Takebe et al., Pharmacology & Therapeutics, 2014, 141: 140-149).

C. Massard et al, "First-in-human study of LY3039478, a Notch signaling inhibitor in advanced or metastatic cancer", Journal of Clinical Oncology, Vol. 33, No. 15, discloses LY3039478 as an orally bioavailable, potent, and highly selective Notch inhibitor (Notch 1-4).

E. Yuen, "Population pharmacokinetics and pharmacodynamics for an oral Notch inhibitor, LY3039478, in the first-in-man study", Cancer Research, discloses LY3039478 as a potent Notch inhibitor that prevents release of the Notch Intracellular Domain (NICO) by inhibiting proteolytic activity of the gamma (y) -secretase complex and thereby decreasing Notch signalling and its downstream biologic effects.

WO2016/168014A1 discloses a medicament comprising 4,4,4-trifluoro-N-[(1S)-2-[[(7S)-5-(2- hydroxyethyl)-6-oxo-7H-pyrido[2,3-d][3]benzazepin-7-yl]amino]-1-methyl-2-oxo- ethyl]butanamide or a pharmaceutically acceptable salt or hydrate thereof for treating leiomyosarcoma.

The skilled artisan will appreciate that a "loading dose" of a drug is an initial higher dose of a drug given at the beginning of a course of treatment before dropping down to a lower "maintenance dose." A loading dose is typically useful for drugs that achieve their therapeutic threshold level relatively slowly. An initial loading dose or doses are administered for the drug to reach the appropriate therapeutic level more rapidly than if administered only at a lower fixed dose.

The present invention balances a need for a therapeutic agent dose regimen that exhibits activity (efficacy) in the treatment of solid tumor cancers while mitigating gastrointestinal toxicity. There is also a need for a dose regimen in which 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide, or a pharmaceutically acceptable salt or hydrate thereof demonstrates therapeutic efficacy and durable response in solid tumor cancer patients without adversely impacting efficacy, or causing dose limiting, or dose schedule limiting gastrointestinal toxicities.

The present invention provides a compound 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide, or a pharmaceutically acceptable salt or hydrate thereof, for use in the treatment of a solid tumor cancer as defined by the claims.
The solid tumor cancer may be selected from the group consisting of triple negative breast cancer, breast cancer, ovarian cancer, melanoma, lung cancer, non-small cell lung cancer, pancreatic cancer, glioblastoma, colorectal cancer, head and neck cancer, cervical cancer, prostate cancer, liver cancer, oral squamous cell carcinoma, skin cancer, medulloblastoma, hepatocellular carcinoma, intrahepatic and extrahepatic cholangiocarcinoma, desmoid tumor, soft tissue sarcoma, leiomyosarcoma, and adenoid cystic carcinoma.

The compound 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide ("Compound 1"), or a pharmaceutically acceptable salt or hydrate thereof, is taught to be a Notch inhibitor in WO 2013/016081.

An "effective amount" means a dose of Compound 1, or pharmaceutically acceptable salt or hydrate thereof, or pharmaceutical composition containing the compound, or pharmaceutically acceptable salt or hydrate thereof, necessary to inhibit Notch signaling in a solid tumor cancer patient, and either destroy the target cancer cells or slow or arrest the progression of the cancer in a patient. An effective amount encompasses both a loading or first dose and a maintenance or second dose of Compound 1, or pharmaceutically acceptable salt or hydrate thereof, or pharmaceutical composition containing the compound, or a pharmaceutically acceptable salt or hydrate thereof, necessary to inhibit Notch signaling in a solid tumor cancer patient, and either destroy the target cancer cells or slow or arrest the progression of the cancer in a patient. Loading doses of Compound 1 or a pharmaceutically acceptable salt or hydrate thereof in an adult patient are in the range of 75 to 150 mg/dose administered twice (two days) in a seven day week or three times (three days) in a seven day week (TIW). Up to 6 loading doses are administered over 14 days of a 28 day cycle. Also preferably, up to 3 loading doses are administered over 7 days of a 28 day cycle. It will be appreciated the number of loading doses is dependent on whether the administration regimen is twice per week or three times per week. A maintenance or second dose of 50 mg per dose is administered TIW following the loading doses. The maintenance dose is administered over the remaining days of the first 28 day cycle to one or more additional 28 day cycles.

During administration of the loading dose, 1-80 mg/day of a corticosteroid is administered.

The terms "treatment," "treat," and "treating," are meant to include the full spectrum of intervention for the solid tumor cancer, such as administration of the active compound to alleviate, to slow, or reverse one or more of the symptoms, and to delay progression of the cancer even if the cancer is not actually eliminated.

The "gastrointestinal toxicities" the present dose regimen (loading dose administration followed by maintenance dose administration and the optional administration of a corticosteroid) may ameliorate or mitigate include diarrhea, nausea, vomiting, mucoid enteropathy and/or colitis. Higher doses, more frequent dosing, and more weeks, or cycles of treatment tend to cause higher grades of gastrointestinal toxicities in patients. Mitigating or ameliorating these toxicities may facilitate a patient receiving additional doses, and/or weeks or cycles of treatment for their cancer.

"Corticosteroids" means hydrocortisone, hydrocortisone acetate, cortisone acetate, tixocortol pivalate, prednisolone, methylprednisolone, and prednisone, preferably prednisone. Although a dose of 1-50 mg/day is contemplated, it is possible to increase the dose up to 80 mg/day.

As used herein, the term "patient" means mammal; "mammal" means the Mammalia class of higher vertebrates; and the term "mammal" includes, but is not limited to, a human.

The solid tumor cancers against which the preset dosing regimen will be efficacious while mitigating gastrointestinal toxicities include triple negative breast cancer, breast cancer, ovarian cancer, melanoma, lung cancer, non-small cell lung cancer, pancreatic cancer, glioblastoma, colorectal cancer, head and neck cancer, cervical cancer, prostate cancer, liver cancer, oral squamous cell carcinoma, skin cancer, medulloblastoma, hepatocellular carcinoma, intrahepatic and extrahepatic cholangiocarcinoma, desmoid tumor, soft tissue sarcoma, leiomyosarcoma, and adenoid cystic carcinoma.

In the present invention a loading dose (first dose) administration of up to 6 doses of 75 to 150 mg/dose twice (two days) in a seven day week or three times (three days) in a seven day week (TIW) during 14 days of a 28 day cycle is used. The number of doses a patient receives may be adjusted to provide a more optimal therapeutic benefit to a patient and/or to mitigate or ameliorate observed toxicities or symptoms related to tumor lysis syndrome. A maintenance dose (second dose) administration of 50 mg per dose TIW is preferred for the remaining days of a first 28 day cycle and may be extended to one or more additional 28-day cycles. The maintenance dose, or second dose, is administered through one or more partial or full 28 day cycles at the discretion of a physician. Administration (pre-, concomitant, or post-administration of Compound 1 or a pharmaceutically acceptable salt or hydrate thereof) of a corticosteroid, and most preferably prednisone, during the loading dose administration of Compound 1 to mitigate or ameliorate gastrointestinal toxicities is contemplated.

The skilled artisan will appreciate the dose regimen of the present invention is provided to afford a more optimal therapeutic efficacy, while ameliorating or mitigating gastrointestinal toxicities. This regimen is in contrast to administering a fixed dose with dose or administration adjustments by, and at the discretion of, a physician that are known and routinely used such as for patients with renal or hepatic impairment or to mitigate toxicities based on individual patient variabilities and response to the active pharmaceutical agent.

The compound of the present invention is preferably formulated as a pharmaceutical composition using a pharmaceutically acceptable carrier and administered by a variety of routes. Preferably, such compositions are for oral administration. Such pharmaceutical compositions and processes for preparing them are well known in the art.

(*See, e.g.*, Remington: The Science and Practice of Pharmacy, L. V. Allen, Editor, 22nd Edition, Pharmaceutical Press, 2012). In a particular embodiment, the pharmaceutical composition comprises 4,4,4-trifluoro-N-[(1S)-2-[[(7S)-5-(2-hydroxyethyl)-6-oxo-7H-pyrido[2,3-d][3]benzoazepin-7-yl]amino]-1-methyl-2-oxo-ethyl]butanamide, or a pharmaceutically acceptable salt or hydrate thereof, and a pharmaceutically acceptable carrier.

The compound of the present invention is capable of reaction with a number of inorganic and organic acids to form pharmaceutically acceptable acid addition salts. Such pharmaceutically acceptable salts and common methodology for preparing them are well known in the art. *See, e.g.,* P. Stahl, et al., HANDBOOK OF PHARMACEUTICAL SALTS: PROPERTIES, SELECTION AND USE, (VCHA/Wiley-VCH, 2002); S.M. Berge, et al., "Pharmaceutical Salts, "Journal of Pharmaceutical Sciences, Vol. 66, No. 1, January 1977.

Compound 1, or a pharmaceutically acceptable salt or hydrate thereof, may be prepared by a variety of procedures known in the art, as well as those described in WO 2013/016081. The specific synthetic steps may be combined in different ways to prepare Compound 1, or a pharmaceutically acceptable salt or hydrate thereof.

Compound 1 is named 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide; and may also be named: N-[(1S)-2-[[(7S)-6,7-dihydro-5-(2-hydroxyethyl)-6-oxo-5H-pyrido[3,2-a][3]benzazepin-7-yl]amino]-1-methyl-2-oxoethyl]-4,4,4-trifluorobutanamide; other names may be used to unambiguously identify Compound 1.

Compound 1 is named as a single stereoisomer. There are two chiral centers giving rise to four stereoisomers. As used herein, references to Compound 1 are meant to also include stereoisomeric mixtures including Compound 1. Herein, the Cahn-Ingold-Prelog designations of (R)- and (S)- are used to refer to specific isomers. Specific stereoisomers can be prepared by stereospecific synthesis using enantiomerically pure or enriched starting materials. The specific stereoisomers of starting materials, intermediates, or racemic mixtures including Compound 1 can be resolved by techniques well known in the art, such as those found in Stereochemistry of Organic Compounds, E. I. Eliel and S. H. Wilen (Wiley 1994) and Enantiomers, Racemates, and Resolutions, J., Jacques, A. Collet, and S. H. Wilen (Wiley 1991), including chromatography on chiral stationary phases, enzymatic resolutions, or fractional crystallization or chromatography of diastereomers formed for that purpose, such as diastereomeric salts. While all mixtures containing the compound of the present invention are contemplated within the present invention, the preferred embodiment is Compound 1.

It has also been found that Compound 1 exists as atropisomers, or specific conformers. In aqueous solutions, 8-9% of atropisomer 2 (minor atropisomer) is detected by ¹H NMR and LC-MS in equilibrium with atropisomer 1 (major atropisomer) at ambient temperature after 24 hours. In organic solvents, at ambient temperature after 24 hours, approximately 1-2% of atropisomer 2 is detected by ¹H NMR and LC-MS in equilibrium with atropisomer 1. Although detectable by ¹H NMR and LC-MS analysis, atropisomer 2 is not isolable.

The compounds employed as initial starting materials in the synthesis of the compound of the present invention are well known and, to the extent not commercially available, are readily synthesized using specific references provided, by standard procedures commonly employed by those of ordinary skill in the art or are found in general reference texts.

Examples of known procedures and methods include those described in general reference texts such as Comprehensive Organic Transformations, VCH Publishers Inc, 1989; Compendium of Organic Synthetic Methods, Volumes 1-10, 1974-2002, Wiley Interscience; Advanced Organic Chemistry, Reactions Mechanisms, and Structure, 5th Edition, Michael B. Smith and Jerry March, Wiley Interscience, 2001; Advanced Organic Chemistry, 4th Edition, Part B, Reactions and Synthesis, Francis A. Carey and Richard J. Sundberg, Kluwer Academic / Plenum Publishers, 2000, etc., and references cited therein.

Cancer is increasingly recognized as a heterogeneous collection of diseases whose initiation and progression are induced by the aberrant function of one or more genes that regulate DNA repair, genome stability, cell proliferation, cell death, adhesion, angiogenesis, invasion, and metastasis in cell and tissue microenvironments. Variant or aberrant function of the "cancer" genes may result from naturally occurring DNA polymorphism, changes in genome copy number (through amplification, deletion, chromosome loss, or duplication), changes in gene and chromosome structure (through chromosomal translocation, inversion, or other rearrangement that leads to deregulated gene expression), and point mutations. Cancerous neoplasms may be induced by one aberrant gene function, and maintained by the same aberrant gene function, or maintenance and progression exacerbated by additional aberrant gene functions.

Beyond the genetic chromosomal aberrations mentioned above, each of the cancers may also include epigenetic modifications of the genome including DNA methylation, genomic imprinting, and histone modification by acetylation, methylation, or phosphorylation. An epigenetic modification may play a role in the induction and/or maintenance of the malignancy.

Diagnosis of cancerous malignancies by biopsy, immunophenotyping and other tests are known and routinely used. In addition to high resolution chromosome banding and advanced chromosomal imaging technologies, chromosome aberrations in suspected cases of cancer can be determined through cytogenetic analysis such as fluorescence in situ hybridization (FISH), karyotyping, spectral karyotyping (SKY), multiplex FISH (M-FISH), comparative genomic hybridization (CGH), single nucleotide polymorphism arrays (SNP Chips) and other diagnostic and analysis tests known and used by those skilled in the art.

PET/CT imaging of cancer with combined positron emission tomography (PET) and X-ray computerized tomography (CT) scanners has become a standard component of diagnosis and staging in oncology. The use of the radiolabeled tracer 2-deoxy-2-[¹⁸F]fluoro-D-glucose (FDG) is used for the majority of all PET/CT imaging procedures. One of the advantages of PET/CT imaging is its ability to detect, very early during treatment, significant changes in glucose metabolism or even complete shutoff of the neoplastic cell metabolism as a surrogate of tumor chemosensitivity assessment. In addition to cancer detection and staging, PET/CT imaging is becoming increasingly important as a quantitative monitor of individual response to therapy and an evaluation tool for new drug therapies. Changes in FDG accumulation have been shown to be useful as an imaging marker for assessing response to therapy. RECIST criteria, where response of tumors to therapy has traditionally assessed by measurement of changes in size/dimension of the tumors in CT images may not evidence early response to the therapy. Changes in size of tumors as a result of therapy may take a long period of time to develop. The most widely used parameter is the standardized uptake value (SUV) is defined as the maximal SUV value (SUV_{MAX}) in the region of interest and reduction in SUV_{MAX} is generally considered the most reliable indicator of the metabolic activity shutdown.

Aberrant constitutive Notch signaling is implicated in a number of solid tumor malignancies (cancers) including breast cancer, ovarian cancer (Park et al. Cancer Research, 2006(66):6312-6318), melanoma (Gast et al. Genes, Chromosomes & Cancer, 2010(49):733-745), lung cancer, non-small cell lung cancer (Westhoff et al. PNAS, 2009(106):22293-22298), pancreatic cancer, glioblastoma, colorectal cancer, head and neck cancer, cervical cancer, prostate cancer, liver cancer, squamous cell carcinoma (oral), skin cancer and medulloblastoma (Ranganathan et al., Nature Review Cancer, 2011(11):338-351 and Supplementary Information S1 (table)). Aberrant Notch signaling may be activated in particular soft tissue sarcomas Guijarro et al. Am J Pathol, 2013(182(6)):2015-2027.

### Preclinical Evaluations

A study is carried out to assess the toxicity and toxicokinetics of 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide in rats given oral gavage doses of 0, 1.3, or 4.3 mg/kg once daily for 2 weeks or 1, 3, 10, or 30 mg/kg 3 times/week for 2 weeks.

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide is combined with a vehicle comprising 1% carboxymethylcellulose sodium, 0.25% polysorbate 80 and 0.05% Dow Corning^{®} Antifoam 1510-US in purified water. Sprague Dawley CD^{®}/IGS rats (crl:CD(SD)), International Genetic Standardization (IGS) 7-9 week old rats, 3/sex/group, Charles River Laboratories, Inc, are used for the study. Animals are fed *ad libitum* with 12 hours light, 12 hours dark cycle maintained in ventilated stainless-steel racks at an ambient temperature of 22.2 +/- 8° C and relative humidity of 30% to 70%. The test compound is dosed according to Table 1. The test compound is administered by oral gavage in a volume of 10 mL/kg body weight. The duration of the study for all groups is 14 days.

**Table 1. Dosing Regimen**

| Frequency | Dose (mg/kg) | Animal Group(s) |
|---|---|---|
| Daily | 0 | 01 |
| Daily | 1.3 | 06 & 12 |
| Daily | 4.3 | 07 & 13 |
| 3 times/week | 1 | 02 & 08 |
| 3 times/week | 3 | 03 & 09 |
| 3 times/week | 10 | 04 & 10 |
| 3 times/week | 30 | 05 & 11 |

Observations, at a minimum, for Groups 01, 06 and 07 are recorded daily before dosing, approximately one (1) hour postdose, and in the afternoon except afternoon observations are not recorded on weekends. Observations for Groups 02, 03, 04, and 05 are recorded beginning on Day 3. At a minimum, observations are recorded as described on dosing days. On non-dosing days, observations are recorded at approximately the same time as for groups receiving daily doses. Animals are weighed on Days 1, 3, 6, 10, and 14. Terminal body weights are collected at scheduled necropsy. Food consumption weights are recorded on Days 1, 3, 6, 10, and 14.

Additional Groups of animals 3/sex/group (Groups 08, 09, and 12) and 3 females/group (Groups 10, 11, and 13) are dosed for toxicokinetic evaluations. For Groups 08, 09, and 10, blood is collected from each animal at the following times relative to dosing on Days 3 and 14: 0 (predose; Day 14 only), 0.5, 1, 2, 4, 8, 24, 30, and 48 hours postdose. For Group 11, blood is collected from each animal at the following times relative to dosing on Day 3: 0.5, 1, 2, 4, 8, 24, 30, and 48 hours postdose. For Group 12, blood is collected from each animal at the following times relative to dosing on Days 1 and 14: 0 (predose; Day 14 only), 0.5, 1, 2, 4, 8, and 24 hours postdose. For Group 13 blood is collected from each animal at the following times relative to dosing on Day 1: 0.5, 1, 2, 4, 8, and 24 hours postdose. Groups 08 through 13 are used for toxicokinetic evaluations. Among other evaluations, the blood samples are used for pharmacokinetic parameters of absorption, distribution, metabolism, and excretion analysis.

Exposure (AUC₀₋₂₄ₕᵣ₎ increased in a roughly linear and dose-proportional manner following both single and multiple doses. No accumulation or major differences between sexes are identified. Due to morbidity and/or mortality, exposure data following multiple doses was only available for a limited number of animals at dose levels of 1.3 mg/kg daily and 10 mg/kg given 3 times/week and was not available at dose levels of 4.3 mg/kg daily or 30 mg/kg given 3 times/week. Changes in body weight and food consumption parameters are observed at dose levels of 1.3 and 4.3 mg/kg administered daily and at dose levels of 10 and 30 mg/kg administered 3 times/week. Substantial body weight loss is observed at dose levels of 4.3 mg/kg given daily or 30 mg/kg given 3 times/week. At dose levels of 1.3 mg/kg given daily or 10 mg/kg given 3 times/week, mean body weights are generally similar to or greater than pretreatment but were slightly to moderately decreased relative to the control group. Minimal to severe decreases in food consumption are also noted and correlated with the decreased body weight gain and/or body weight loss.

All non-toxicokinetic animals given 30 mg/kg 3 times/week are euthanized on Day 10 due to poor condition. Two of three toxicokinetic (TK) animals given 10 mg/kg 3 times/week are euthanized on Day 10 due to poor condition and the remaining TK animal was found dead prior to the 48 hr TK time point on Day 16. TK animals given daily doses of 4.3 mg/kg are found dead on Days 7, 8, or 10, and animals given daily doses of 4.3 mg/kg are euthanized on Day 10 due to poor condition. In addition, 2 of 3 female TK animals given daily doses of 1.3 mg/kg are euthanized on Day 10 due to poor condition. All non-TK animals, male TK animals, and 1 of 3 female TK animals given daily doses of 1.3 mg/kg survived until the scheduled study termination. All non-TK animals given ≤10 mg/kg and TK animals given ≤3 mg/kg 3 times/week survived until the scheduled study termination. The poor condition requiring euthanization is attributed to gastrointestinal toxicity, although inflammation, decreased food consumption, stress, and dehydration are also observed. Compound related toxicities are seen in non-TK animals of both sexes given 10 mg/kg administered 3 times/week, females given 3 mg/kg 3 times/week and are limited to mucoid enteropathy involving multiple levels of the intestinal tract and minimal to slight renal tubular degeneration in males given 10 mg/kg 3 times/week.

Rats receiving the same total weekly dose (1.3 mg./kg daily or 3 mg/kg three times/week; and 4.3 mg/kg daily or 10 mg/kg three time/week) had more severe gastrointestinal toxicity with daily dosing compared to three times/week dosing in this study.

A study is carried out to assess the Toxicity and Toxicokinetics of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide in dogs given oral gavage doses of 0 or 1.3 mg/kg once daily for 6 days or 0.3 or 3 mg/kg every other day for a total of 3 doses.

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide is combined with a vehicle comprising 1% carboxymethylcellulose sodium, 0.25% polysorbate 80 and 0.05% Dow Corning^{®} Antifoam 1510-US in purified water. Dogs (beagle), Covance Research Products, 1 animal/sex/group are fed daily (soon after dosing) and are fasted overnight prior to scheduled chemistry blood collections and necropsy. The test compound is administered by oral gavage in a volume of 2 mL/kg body weight. The duration of the study for all groups is 6 days.

**Table 2 Dosing Regimen**

| Frequency | Dose (mg/kg) | Animal Group(s) |
|---|---|---|
| Daily | 0 | 01 |
| Daily | 1.3 | 04 |
| 3 times/week; Days 2, 4, and 6 | 0.3 | 02 |
| 3 times/week Days 2, 4, and 6 | 3.0 | 03 |

### Study Parameters

### Toxicokinetics

Sample Collection:
Groups 01 and 04, Blood is collected at the following times relative to dosing on Day 1: 0.5, 1, 2, 4, 8, and 24 hours postdose.
Groups 02 and 04: Blood is collected at the following times relative to dosing on Days 2 and 6: 0 (predose; Day 6 only), 0.5, 1, 2, 4, 8, 24, 30, and 48 hours postdose.

### Metabolite Analysis

Sample Collection: Blood is collected from one animal/sex in Group 03 at 2 hours postdose on Day 6.

### Observations

Groups 01 and 04: At a minimum, observations are recorded daily before dosing, approximately 1 hour postdose, and in the afternoon, with the following exception: afternoon clinical observations are not recorded on weekends.
Groups 02 and 03: At a minimum, observations are recorded before dosing,
   approximately 1 hour postdose, and in the afternoon on Days 2 and 6; before dosing and approximately 1 hour postdose on Day 4; and at approximately the same times as Groups 01 and/or 04 on Days 3, 5, and 7.

### Body Weights:

Animals are weighed on Days 1 and 7.

### Food Consumption:

A qualitative assessment of food consumption is made each day by visual estimation of the amount of food remaining (recorded in increments of 25%).

### Pathology.

### Sample Collection:

Groups 01 and 04: Blood samples are collected before dosing on Day 1 and on Day 7 (not dosed on Day 7).
Groups 02 and 03: Blood samples are collected before dosing on Days 1 and 6.
Urine samples are collected from all animals by cystocentesis at the scheduled necropsy.

Animals given 1.3 mg/kg are euthanized following 6 daily doses due to poor condition. Animals given 0.3 or 3 mg/kg 3 times/week (Days 2, 4, and 6) survived until the scheduled termination. Compound-related signs limited to animals given 6 daily doses of 1.3 mg/kg included lateral recumbency and labored respiration for the female on Day 7, decreased activity for both animals on Days 6 and 7, and dehydration (decreased skin elasticity) for both animals on Day 7. Other symptoms at this dose level included red, dark (female only), mucoid, and watery (female only), feces. Compound-related signs for animals given 3 mg/kg 3 times/week included red (male only), mucoid, and watery (male only), feces. In animals given 0.3 mg/kg 3 times/week, signs are limited to the female and included mucoid, and watery feces.

Body weight loss occurred in all compound-treated groups and is considered adverse in animals given 3 mg/kg 3 times/week or 1.3 mg/kg daily for 6 days. Decreased food consumption also occurred in all compound-treated groups.

Adverse gastrointestinal (GI) changes occurred in animals given 3 mg/kg 3 times weekly and 1.3 mg/kg daily, and resulted in early euthanasia of both animals given 1.3 mg/kg. The male given 0.3 mg/kg 3 times weekly has adverse inflammatory changes in the large intestine and evidence of systemic inflammation that are likely compound related, but lacked the more characteristic mucoid enteropathy signs.

Gastrointestinal changes occurred at all levels of the GI tract and included mucoid epithelial changes (mucoid enteropathy), erosion, ulceration, and inflammation involving the submucosa and deeper layers of the intestinal wall. Intestinal changes are most pronounced in animals given 1.3 mg/kg daily and included up to marked mucoid enteropathy with red fluid intestinal contents, erosion, ulceration, and mostly neutrophilic mixed inflammation. Mucoid enteropathy is characterized by increased numbers of goblet cells at all levels of the mucosa, and when more pronounced, is associated with disorganization, crypt dilation, and luminal contents composed of mucus and large numbers of neutrophils admixed with exfoliated enterocytes and cellular debris.

**Table 3 Mean TK parameters in Beagle dogs (one male, one female, n = 2) following a single oral dose or multiple TIW oral doses (3 doses/week) of 0.3 or 3 mg/kg.**

| Single oral dose | | | |
|---|---|---|---|
| Parameter | Units | 0.3 mpk | 3 mpk |
| AUC | ng*Hours/mL | 1290 | 5860 |
| Cmax | ng/mL | 309 | 2730 |
| Tmax | Hours | 0.500 | 0.500 |

| Multiple oral doses (3 doses/week) | | | |
|---|---|---|---|
| Parameter | Units | 0.3 mpk | 3 mpk |
| AUC | ng*Hours/mL | 3070 | 14400 |
| Cmax | ng/mL | 2460 | 4530 |
| Tmax | Hours | 0.750 | 0.750 |

**Table 4. Mean TK parameters in Beagle dogs (one male, one female, n = 2) following a single oral dose at 1.3 mg/kg**

| Parameter | Units | Subject 0007 M | Subject 0008 F | Mean | n |
|---|---|---|---|---|---|
| AUC | ng*Hours/mL | 4030 | 4500 | 4270 | 2 |
| Cmax | ng/mL | 1800 | 2070 | 1940 | 2 |
| Tmax | Hours | 0.500 | 0.500 | 0.500 | 2 |

Beagle dogs receiving 1.3 mg/kg daily doses are euthanized following 6 doses. Beagle dogs receiving 0.3 or 3 mg/kg three times/week survived until the scheduled necropsy. Dogs receiving daily dosing had more severe gastrointestinal toxicity compared to three times/week dosing (where the daily dosing dogs and the three times/week dogs received the same total weekly dose) in this study.

The preclinical rat and dog data shows that for the same total weekly dose, once daily administration is not tolerated. In both regimens, 24 hour drug concentrations are minimal, but with TIW dosing there is 24-48 hours between doses where there is no drug in the system. These data suggest that some time off drug is required to minimize toxicity.

### In-vivo efficacy and target inhibition studies - Animal studies

To evaluate in vivo efficacy and effect of 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide on inhibition of Notch processing pharmacodynamics (PD), several cell lines- and patient-derived xenograft models are used. A2780 is a human ovarian cell line (Sigma-Aldrich, No. 93112519); SW480 is a human colorectal cell line (ATCC No. CCL-228); HCT 116 is a human colorectal cell line (ATCC No. CCL-247); U-87 MG is a human glioblastoma cell line (ATCC No. HTB-14); A375 is a human malignant melanoma cell line (ATCC No. CRL-1619); K-562 is a human chronic myelogenous leukemia (CML) cell line characterized by the presence of a fusion transcript comprised of the Bcr and Abl1 genes (ATCC No. CCL-243); Jurkat; HEL 92.1.7 is a human erythroleukemia cell line (ATCC No. TIB-180). Each of the cell lines are obtained from the American Type Culture Collection (ATCC) at the ATCC number stated, except the A2780 cell line which is obtained from Sigma-Aldrich at the stated catalog number. The cells are grown in their respective, recommended culture media at 37°C in 5% CO₂ with humidity in the atmosphere. A2780 (2 × 10⁶), SW480 (6 × 10⁶), HCT 116 (6 × 10⁶), U-87 MG (6 × 10⁶), and A-375 (10 × 10⁶) cells in a 1:1 matrigel mix (0.2 mL volume) are implanted by subcutaneous injection in the hind leg of 6-8 weeks of age athymic nude female mice (Harlan Laboratories). K-562 (6 × 10⁶) cells in a 1:1 matrigel mix (0.2 mL volume) are implanted by subcutaneous injection in the hind leg of 6-8 weeks of age CD1 nµ/nµ female mice (Charles River Laboratories). HEL 92.1.7 (7 × 10⁶) in a 1:1 matrigel mix (0.2 mL volume) are implanted by subcutaneous injection in the hind leg of 6-8 weeks of age CB17 severely combined immune deficient female mice (Taconic Farms). Patient-derived tumors are minced into 1-2 mm pieces and mixed with matrigel (1:1) in 0.2 ml volume and implanted by subcutaneous injection in the hind leg of 6-8 weeks of age athymic nude female mice (Harlan Laboratories). Patients-derived tumor models include: human colon carcinoma (EL2144), human triple negative invasive ductal breast carcinoma (EL1997), human colon carcinoma (EL1989, EL 1986), and human glioblastoma (EL 2056) with samples obtained after patient consent and hospital approval from IU Health, Methodist Hospital, Indianapolis, Indiana, USA 46206. A total of 7 to 10 mice are used for each group. Just before implantation for A2780, SW480, HEL 92.1.7, A-375, K-562, and patient-derived tumor models, animals are irradiated (450 Total Body Irradiation). Mice are fed *ad libitum* on normal chow. Treatment is initiated with oral administration (gavage) of compound or vehicle (1% Na-CMC in 0.25% Tween^{®}-80) in 0.2 mL volume when tumor size reached to 150 ± 50 mm³. At designated time points following treatment, animals are sacrificed by CO₂ asphyxiation and cervical dislocation. Tumors are removed and used for PD response analysis. Tumor growth and body weight are monitored over time to evaluate efficacy and signs of toxicity. Bidimensional measurements of tumors are performed twice a week and tumor volumes are calculated based on the following formula: (Tumor Volume) = [(L) × (W2) × (II/6)] where L is mid-axis length and W is mid-axis width. Tumor volume data are transformed to a log scale to equalize variance across time and treatment groups. The log volume data are analyzed with a two-way repeated measures analysis of variance by time and treatment using the MIXED^{™} procedures in SAS^{™} software (version 8.2). The correlation model for the repeated measures is spatial power. Treated groups are compared to the control group at each time point. The MIXED^{™} procedure is also used separately for each treatment group to calculate adjusted means and standard errors at each time point. Both analyses account for the autocorrelation within each animal and the loss of data that occurs when animals with large tumors are removed from the study early. The adjusted means and standard errors are plotted for each treatment group versus time. Antitumor activity is expressed as tumor growth inhibition percentage (TGI %) and is calculated by comparing tumor volume in the treatment group with vehicle treatment group. Percentage Tumor Growth Inhibition (%TGI) and statistical significance value (p value) for4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide is measured essentially as described above and summarized in Table 5.

### N1ICD Analysis

To evaluate N1ICD levels in tumors, approximately 75 mg is cut from the frozen tumor and minced prior to homogenization (actual mass recorded). Frozen tumor samples are transferred to Lysing Matrix-D^{™} tubes and re-suspended in ice-cold XY lysis buffer (25 mM Tris pH 7.5, 10 µg/ml Trypsin/Chymotrypsin inhibitor, 10 µg/ml Aprotinin, 60 mM Beta-glycerol phosphate , 1% Triton^{®} X-100, 10 mM NaF, 2.5 mM pyrophosphate, 150 mM NaCl, 15 mM ethylene diamine tetra acetic acid (EDTA) pH 8.0, 5 mM ethylene glycol-bis(2-aminoethylether)-N,N,N',N'-tetra acetic acid (EGTA) pH 8.0, 1 mM Na Vanadate, 10 µg/ml Leupeptin, 1 mM dithiothreitol, 1 µM microcystin LR, 10 µg/ml N-p-tosyl-L-phenylalanine chloromethyl ketone (TPCK), 2 mM Nα-p-tosyl-L-arginine methyl ester hydrochloride (TAME), 15 mM 4-nitrophenyl phosphate di(tris) salt (PNPP), 0.1 mM 4-(2-aminoethyl) benzenesulfonyl fluoride hydrochloride (AEBSF), 5 mM benzamidine, 1 µM Okadaic Acid) containing 1X Complete tablet (Roche Complete^{™} No. 11697 498 001) and 1X Protease Inhibitor cocktail (Sigma-Aldrich P8340) at a mass: volume ratio of 75 mg/ml buffer. Tissues are homogenized in a Fast Prep FP120 homogenizer (Thermo Scientific, Rockford, IL) at a speed of 6.0 for 30 seconds at 4°C, followed by 15 minute incubation on ice. This is repeated for a total of 2-3 cycles until homogenization is complete. Lysates are spun in a 4°C Eppendorf centrifuge at 30,000 rpm for 15 minutes to remove debris. 400 µl of supernatant is removed and transferred to a new Eppendorf tube and subjected to a freeze/thaw cycle. Samples are re-spun in a 4°C Eppendorf centrifuge at 30,000 rpm for 30 minutes and 120 µl of supernatant is collected for analysis. Total protein concentration is determined using Pierce BCA Protein Assay Kit^{™} (Thermo Scientific, Rockford, IL) using a Thermomax^{™} plate reader (Molecular Devices, Sunnyvale, CA). N1ICD levels are determined using a custom N1ICD ELISA. Analyte is captured with a cleaved Notch1(Val1744)-specific custom rabbit monoclonal antibody and detected with a C-terminal Notch1 SULFO-TAG^{™} (Meso Scale Discovery, Gaithersburg, Maryland) polyclonal sheep antibody (R&D Systems, Minneapolis, MN). Lysates are diluted to 2 µg/µl in ice-cold ELISA tris lysis buffer (R6OTX) (Meso Scale Discovery, Gaithersburg, Maryland) containing 1X Complete tablet (Roche Complete^{™} mini No. 11 836 153 001) and 1X Protease Inhibitor cocktail (Sigma-Aldrich P8340), and 25µl is added to the ELISA plate. Incubation of 50 µg protein lysate is done at RT for one hour each to capture analyte and with detection antibody. Plates are read on a Sector Imager 6000^{™} (Meso Scale Discovery, Gaithersburg, Maryland). Background subtracted N1ICD is normalized to total protein and presented as % inhibition relative to the vehicle-treated group. N1ICD % inhibition and statistical significance (p value) as measured by Dunett's method in tumors harvested 4 hours after last dose for 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide is analyzed essentially as described above and summarized in Table 5.

**Table 5.**

| Tumor Model | Dose (mg/kg) | Schedule | % TGI (p Value) | % N1ICD Inhibition (p Value) |
|---|---|---|---|---|
| A2780 | 10 | Q2Dx11 | 56.55 (< 0.0001) | 68.5 (< 0.0001) |
| A2780 | 10 | Q3Dx8 | 32.99 (< 0.0001) | 55.3 (< 0.0001) |
| A2780 | 3 | (BID)QDx7+(BID)Q2Dx7 | 72.35 (< 0.0001) | 50.7 (0.0004) |
| A2780 | 3 | QDx21 | 46.60 (< 0.0001) | 62.8 (< 0.0001) |
| A2780 | 10 | Q2Dx13 | 51.11 (< 0.0001) | 74.8 (< 0.0001) |
| A2780 | 8 | Q2Dx13 | 68.60 (< 0.0001) | 71.7 (< 0.0001) |
| A2780 | 7 | Q2Dx13 | 56.95 (< 0.0001) | 65.9 (< 0.0001) |
| A2780 | 6 | Q2Dx13 | 36.33 (< 0.05 to < 0.01) | 60.7 (< 0.0001) |
| A2780 | 3 | Q2Dx13 | 36.65 (< 0.05 to < 0.01) | 58.6 (< 0.0001) |
| A2780 | 1.5 | QDx26 | 33.36 (< 0.05 to < 0.01) | 59.0 (< 0.0001) |
| SW480 | 8 | (Mon, Wed, Fri)x5 | 61.00 (< 0.0001) | 72.5 (= 0.0002) |
| HCT 116 | 8 | (Mon, Wed, Fri)x4 | 37.58 (< 0.05 to < 0.01) | 73.0 (= 0.0005) |
| U-87 MG | 8 | (Mon, Wed, Fri)x4 | 53.33 (< 0.0001) | 87.8 (< 0.0001) |
| A-375 | 8 | (Mon, Wed, Fri)x4 | 28.47 (NS) | 77.5 (< 0.0001) |
| K-562 | 8 | (Mon, Wed, Fri)x4 | 54.96 (< 0.01 to < 0.001) | 47.6 (< 0.0001) |
| HEL 92.1.7 | 8 | Q2dx14 | 7.20 (NS) | 56.7 (< 0.0001) |
| EL1997 | 10 8 | (Q2Dx7), 11-days OFF, (Mon, Wed, Fri)x4 | 80.28 (< 0.0001) | 67.9 (< 0.0001) |
| EL1989 | 10 8 | (Q2Dx7), 11-days OFF, (Mon, Wed, Fri)x3 | 70.42 (< 0.0001) | 79.2 (< 0.0001) |
| EL2144 | 10 | Q2Dx8 | 53.37 (< 0.0001) | ND* |
| EL2056 | 8 | (Mon, Wed, Fri)x5 | 59.05 (< 0.0001) | 83.5 (< 0.0001) |
| EL1986 | 8 | (Mon, Wed, Fri)x5 | 62.00 (< 0.0001) | 84.9 (< 0.0001) |

| | | | | |
|---|---|---|---|---|
| * Not Determined | | | | |

The data in Table 5 show the tumor growth inhibition, and the inhibition of N1ICD cleavage by 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide in various xenograft models of human tumors. The data in Table 5 also shows the effects of alternative dosing regimens on tumor growth inhibition by 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide. The data also shows that more frequent dosing affords greater efficacy.

Using the preclinical data and pharmacokinetic/pharmacodynamic data from patients in a phase 1 dose escalation study, PK/PD models are developed to simulate various dosing regimens in patients, with a goal to maximize the time above 50% inhibition of gene expression and time off drug based on average patient responses.

### Clinical Evaluation

A study of 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate in patients with advanced or metastatic solid tumor cancer.

### Study Design

This study is a multicenter, nonrandomized, open-label, dose-escalation study followed by cohort expansion of oral dosed 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide hydrate in outpatients with advanced or metastatic solid tumor cancer.

### Study Objectives

The primary objective of this study is to determine a recommended Phase 2 dose of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate that may be safely administered to patients according to 2 alternative dosing schedules with co-administration of prednisone and to document antitumor activity..

The secondary objectives of the study are to characterize the safety and toxicity profile of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate as assessed by National Cancer Institute's (NCI) Common Terminology Criteria for Adverse Events (CTCAE) v4.0; to estimate the pharmacokinetic (PK) parameters of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate; and to document any antitumor activity observed with 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate.

Exploratory objectives are to explore renal clearance and PK metabolites of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate in plasma and urine; explore predictive biomarkers related to 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate; explore pharmacodynamic (PD) effects of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide hydrate on biomarkers indicative of Notch activity (Notch intracellular domain by immunohistochemistry or an alternative validated method) including cytokeratin 18 or Rules Based Medicine; explore the utility of positron emission tomography (PET) scan or PET/CT to assess treatment effect with 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate; explore the utility of dynamic contrast enhanced magnetic resonance imaging (DCE-MRI) to assess treatment effect with 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate; and explore the utility of Dynamic Contrast-Enhanced Ultrasonography DCE-US.

### Trial Drug

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate, given orally as capsules 3 times per week for a 28-day cycle or twice per week for 14 days followed by TIW dosing for weeks 3 and 4 of Cycle 1 and TIW for Cycle 2 and beyond for a 28-day cycle.

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate will be supplied as 25 and 50 mg capsules in bottles for oral consumption. These capsules should be stored at room temperature within the temperature range stated on the label.

Prednisone will either be provided or obtained locally as appropriate and required and administered daily at a dose of 20 mg on days 1 through 14, and may be extended through day 28 of Cycle 1.

### Planned Duration of Treatment

Patients will receive 1 cycle (28 days) of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide hydrate unless one or more of the criteria for discontinuation are fulfilled. A patient may receive more than 1 cycle of treatment only if: 1) none of the criteria for discontinuation have been fulfilled, and 2) the investigator determines that the patient is experiencing clinical benefit from the treatment.

The planned duration is not fixed; patients will remain on study until they fulfill one (1) of the criteria for study discontinuation. The post-discontinuation follow-up period begins the day after the patient and the investigator agree that the patient will no longer continue study treatment and is defined by the following periods: The short term follow-up period begins 1 day after discontinuation of study treatment and lasts approximately 30 days. The long-term follow-up period begins 1 day after the short-term follow-up period is completed and continues until death or study closure to collect survival data. After discontinuation, tumor measurements and other study procedures will be performed.

This study will be considered closed approximately 12 months from the date that the last patient was enrolled. Patients who are benefitting from treatment may continue to receive study drug for long-term durations, even after the study has closed and final database lock has occurred in the continued access period.

### Dosing

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate will be administered orally following one of the following schedules (decision at investigator's discretion):
4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate will be administered orally TIW following 1 of these schedules (decision at investigator's discretion):
Monday, Wednesday, Friday every week for a 28-day cycle
Tuesday, Thursday, Saturday every week for a 28-day cycle
Wednesday, Friday, Sunday every week for a 28-day cycle
Thursday, Saturday, Monday every week for a 28-day cycle

4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate will be administered orally twice a week for 2 weeks in Cycle 1, followed by TIW dosing, following 1 of these schedules (decision at investigator's discretion):
For Cycle 1: Monday and Friday for Weeks 1 and 2, followed by Monday, Wednesday, Friday for Weeks 3 and 4. For Cycle 2 and beyond: Monday, Wednesday and Friday every week for a 28-day cycle.
For Cycle 1: Tuesday and Saturday for Weeks 1 and 2, followed by Tuesday, Thursday, Saturday for Weeks 3 and 4. For Cycle 2 and beyond: Tuesday, Thursday, Saturday every week for a 28-day cycle.
For Cycle 1: Wednesday and Sunday for Weeks 1 and 2, followed by Wednesday, Friday, Sunday for Weeks 3 and 4. For Cycle 2 and beyond: Wednesday, Friday, Sunday every week for a 28-day cycle.
For Cycle 1: Thursday and Monday for Weeks 1 and 2, followed by Thursday, Saturday, Monday for Weeks 3 and 4. For Cycle 2 and beyond: Thursday, Saturday, Monday every week for a 28-day cycle.
Prednisone will be administered daily on Days 1 through 14 of Cycle 1 at the dosage of 20 mg.

### Dose Escalation Phase

Dose escalation will be driven by safety using the 3+3 method.

**Table 6 Proposed Dose-Escalation Scheme**

| **Dose Level** | **Loading Dose (mg)** |
|---|---|
| 1 | 75 |
| 2 | 100 |
| 3 | 125 |
| 4 | 150 |

By nature of being a dose-escalation study, data will be evaluated on an ongoing basis until the maximum tolerated dose (MTD) is determined. If the MTD has not yet been achieved at the highest pre-specified dose level, based on both safety and the available PK data, following discussion with investigators, additional dose levels may be investigated.

Once the MTD has been defined, the cohort-expansion phase will be opened.

This study will explore 2 alternate dosing schedules and once an MTD has been defined for each of these alternate schedules, a cohort expansion in approximately 15 leiomyosarcoma patients with histological evidence of non-resectable or metastatic leiomyosarcoma with prescreened, immunohistochemistry (IHC), alterations in the Notch pathway such as mutations, amplification, or gene expressions related to the Notch pathway will be opened for each dosing schedule.

### Criteria for Evaluation

Safety: NCI CTCAE, version 4.0, adverse events (AE) and dose-limiting toxicities (DLT); collection of blood and urine samples for standard laboratory tests, including chemistry, hematology, coagulation, and urinalysis.

Bioanalytical (including PK and PD): Plasma concentrations of 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide hydrate.

### Efficacy:

Efficacy will be assessed using Response Evaluation Criteria in Solid Tumors (RECIST) v1.1 for solid tumors. Each patient will be assessed by 1 or more of the following radiologic tests for tumor measurement: X-ray computerized tomography (CT) scan; magnetic resonance imaging (MRI); chest X-ray; positron emission tomography (PET) scan; dynamic contrast enhanced-magnetic resonance imaging (DCE-MRI); PET/CT imaging Standardized Uptake Values (SUV_{MAX}); Dynamic Contrast-Enhanced Ultrasonography (DCE-US).

Each patient's full extent of disease will also be assessed with: applicable tumor measurement by RECIST 1.1 (Eisenhauer et al., Eur J Cancer. 2009, 45(2): 228-247); and Choi et al., J Clin Oncol. 2007, 25(13): 1753-1759; and evaluation of performance status by ECOG, Oken et al., Am J Clin Oncol. 1982, 5: 649-655.. To confirm objective responses, all lesions should be radiologically assessed, and the same radiologic method used for the initial response determination should be repeated at least 4 weeks following the initial observation of an objective response, using the sample method that was used at baseline. Partial metabolic response by PET scan is defined as a minimum of 15±25% in tumor [18F]-FDG SUV after one cycle of therapy, and greater than 25% after more than one treatment cycle and should be confirmed at least 4 weeks later, according to PET response criteria of the European Organization for Research and Treatment of Cancer (Young et al., Eur J Cancer, 1999, Dec, 35(13): 1773-82.

### Statistical Methods

Safety: Dose escalation will be driven by safety using the 3+3 method. Model-based analyses that incorporate prior expectations about the dose-toxicity curve will be fitted to the data at the end of each cohort, which will be used by investigators and Lilly clinical research physician to determine the next dose level. The maximum tolerated dose is defined as the highest tested dose that has less than 33% probability of causing a DLT during Cycle 1.

Efficacy: Tumor response data will be tabulated and summarized by study part.

Pharmacokinetics: PK parameters for 4,4,4-trifluoro-N-[(1S)-2-[[(7S)-5-(2-hydroxyethyl)-6-oxo-7H-pyrido[2,3-d][3]benzazepin-7-yl]amino]-1-methyl-2-oxoethyl]butanamide hydrate will be analyzed by standard non-compartmental methods of analysis.

Pharmacodynamics: All PD data will be assessed. Exploratory PK/PD analyses may be conducted to identify the exposure-biomarker response relationship.

Exploratory Samples: Blood samples will be collected for exploratory analysis of circulating Amyloid beta (Aβ) peptides before and after treatment. A mandatory tumor tissue sample and a skin punch sample obtained previously, within two years of the date of enrollment, or a fresh sample if no archival sample can be located for measuring various biomarkers, potentially including gene-expression profiling as well as other exploratory biomarkers. Pre- and post-dose tumor and skin biopsies will also be collected for analysis.

## Claims

1. A compound 4,4,4-Trifluoro-N-[(1S)-2-[[(7S)-5-(2-hydroxyethyl)-6-oxo-7H-pyrido[2,3-d][3]benzazepin-7-yl]amino]-1-methyl-2-oxo-ethyl]butanamide, or a pharmaceutically acceptable salt or hydrate thereof, for use in the treatment of a solid tumor cancer, wherein said treatment comprises administering:
a. loading doses of said compound or a pharmaceutically acceptable salt or hydrate thereof at 75-150 mg/dose, wherein said loading doses are administered twice or three times per week, wherein up to 6 loading doses are administered over 14 days of one 28 day cycle; followed by
b. a maintenance dose administration of 50 mg/dose administered three times per week for the remaining days of the first 28 day cycle,
wherein an adult patient is being treated,
wherein the use further comprises administering, during administration of the loading dose, 1-80 mg/day of a corticosteroid.

2. The compound for use in the treatment of claim 1, wherein the corticosteroid is prednisone.

3. The compound for use in the treatment of claim 1 or claim 2, wherein said loading dose is up to 3 doses over 7 days of the 28 day cycle.

4. The compound for use in the treatment of any one of claims 1-3, wherein said maintenance dose is administered over any remaining days of the 28 day cycle and over one or more additional 28 day cycles.

5. The compound for use in the treatment of any one of claims 1-4, wherein the solid tumor is selected from the group consisting of triple negative breast cancer, breast cancer, ovarian cancer, melanoma, lung cancer, non-small cell lung cancer, pancreatic cancer, glioblastoma, colorectal cancer, head and neck cancer, cervical cancer, prostate cancer, liver cancer, oral squamous cell carcinoma, skin cancer, medulloblastoma, hepatocellular carcinoma, intrahepatic and extrahepatic cholangiocarcinoma, desmoid tumor, soft tissue sarcoma, leiomyosarcoma, and adenoid cystic carcinoma.

## Patentansprüche

1. Verbindung 4,4,4-Trifluor-N-[(1S)-2-[[(7S)-5-(2-hydroxyethyl)-6-oxo-7H-pyrido[2,3-d][3]benzazepin-7-yl]amino]-1-methyl-2-oxoethyl]butanamid oder ein pharmazeutisch unbedenkliches Salz oder Hydrat davon zur Verwendung bei der Behandlung einer Krebserkrankung mit einem soliden Tumor, wobei die Behandlung die Verabreichung von Folgendem umfasst:
a. Aufsättigungsdosen der Verbindung oder eines pharmazeutisch unbedenklichen Salzes oder Hydrats davon in einer Menge von 75-150 mg/Dosis, wobei die Aufsättigungsdosen zwei- oder dreimal pro Woche verabreicht werden, wobei bis zu 6 Aufsättigungsdosen über 14 Tage eines 28-Tage-Zyklus verabreicht werden; gefolgt von
b. einer Erhaltungsdosisverabreichung von 50 mg/Dosis, die dreimal pro Woche für die restlichen Tage des ersten 28-Tage-Zyklus verabreicht wird,
wobei ein erwachsener Patient behandelt wird,
wobei die Verwendung ferner die Verabreichung von 1-80 mg/Tag eines Kortikosteroids während der Verabreichung der Aufsättigungsdosis umfasst.

2. Verbindung zur Verwendung bei der Behandlung nach Anspruch 1, wobei es sich bei dem Kortikosteroid um Prednison handelt.

3. Verbindung zur Verwendung bei der Behandlung nach Anspruch 1 oder Anspruch 2, wobei die Aufsättigungsdosis aus bis zu 3 Dosen über 7 Tage des 28-Tage-Zyklus besteht.

4. Verbindung zur Verwendung bei der Behandlung nach einem der Ansprüche 1-3, wobei die Erhaltungsdosis über verbliebene Tage des 28-Tage-Zyklus und über einen oder mehrere zusätzliche 28-Tage-Zyklen verabreicht wird.

5. Verbindung zur Verwendung bei der Behandlung nach einem der Ansprüche 1-4, wobei der solide Tumor aus der Gruppe bestehend aus dreifach negativem Brustkrebs, Brustkrebs, Eierstockkrebs, Melanom, Lungenkrebs, nichtkleinzelligem Lungenkrebs, Bauchspeicheldrüsenkrebs, Glioblastom, Kolorektalkrebs, Kopf- und Halskrebs, Gebärmutterhalskrebs, Prostatakrebs, Leberkrebs, oralem Plattenepithelkarzinom, Hautkrebs, Medulloblastom, hepatozellulärem Karzinom, intrahepatischem und extrahepatischem Cholangiokarzinom, Desmoidtumor, Weichteilsarkom, Leiomyosarkom und adenoidzystischem Karzinom ausgewählt ist.

## Revendications

1. Composé 4,4,4-trifluoro-N-[(1S)-2-[[(7S)-5-(2-hydroxyéthyl)-6-oxo-7H-pyrido[2,3-d][3]benzazépin-7-yl]amino]-1-méthyl-2-oxo-éthyl]butanamide, ou sel pharmaceutiquement acceptable ou hydrate correspondant, pour une utilisation dans le traitement d'un cancer à tumeur solide, ledit traitement comprenant l'administration :
a. de doses de charge dudit composé ou d'un sel pharmaceutiquement acceptable ou d'un hydrate correspondant à 75-150 mg/dose, lesdites doses de charge étant administrées deux fois ou trois fois par semaine, jusqu'à 6 doses de charge étant administrées sur 14 jours d'un cycle de 28 jours ; suivies par
b. une administration d'une dose de maintenance de 50 mg/dose administrée trois fois par semaine pendant les jours restants du premier cycle de 28 jours,
un patient adulte étant traité,
l'utilisation comprenant en outre l'administration, pendant l'administration de la dose de charge, de 1-80 mg/jour d'un corticostéroïde.

2. Composé pour une utilisation dans le traitement selon la revendication 1, le corticostéroïde étant la prednisone.

3. Composé pour une utilisation dans le traitement selon la revendication 1 ou la revendication 2, ladite dose de charge pouvant atteindre 3 doses sur 7 jours du cycle de 28 jours.

4. Composé pour une utilisation dans le traitement selon l'une quelconque des revendications 1-3, ladite dose de maintenance étant administrée pendant de quelconques jours restants du cycle de 28 jours et sur un ou plusieurs cycles de 28 jours supplémentaires.

5. Composé pour une utilisation dans le traitement selon l'une quelconque des revendications 1-4, la tumeur solide étant sélectionnée dans le groupe constitué par le cancer du sein triple négatif, le cancer du sein, le cancer des ovaires, un mélanome, le cancer du poumon, le cancer du poumon non à petites cellules, le cancer du pancréas, un glioblastome, le cancer colorectal, le cancer de la tête et du cou, le cancer du col de l'utérus, le cancer de la prostate, le cancer du foie, le cancer épidermoïde buccal carcinome, le cancer de la peau, un médulloblastome, un carcinome hépatocellulaire, un cholangiocarcinome intrahépatique et extrahépatique, une tumeur desmoïde, un sarcome des tissus mous, un léiomyosarcome et un carcinome adénoïde kystique.
